# EUROPEAN PATENT APPLICATION

(11) **EP 4 394 038 A1**
(43) Date of publication of application: **03.07.2024**
(21) Application number: 21959080.9
(22) Date of filing: 28.10.2021
(51) Int. Cl.: C12N 15/54, C12N 9/10, C12N 11/14, C12P 17/10, C12P 13/00

(54) **TRANSAMINASE MUTANT AND APPLICATION THEREOF**

(30) Priority: 28.09.2021 CN 202111146751
(71) Applicant: Asymchem Laboratories (Tianjin) Co., Ltd., Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); ZHANG, Na, Tianjin 300457 (CN); VYASA, Williams, Morrisville North Carolina 27560 (US); CUI, Yuxia, Tianjin 300457 (CN); ZHAO, Jiadong, Tianjin 300457 (CN); GAO, Yanyan, Tianjin 300457 (CN); FU, Han, Tianjin 300457 (CN)
(74) Representative: Berggren Oy
(86) International application number: PCT/CN2021/127138
(87) International publication number: WO 2023/050516

(57) **Abstract**

Provided are a transaminase mutant and an application thereof. The transaminase mutant has an amino acid mutation based on a sequence shown in SEQ ID NO: 1, the amino acid mutation being a single position mutation from among W60Y, Y168A, V379W, V379L, V379M, C418Q and C418W or a combination thereof. The activity, stability, and tolerance to temperature, pH and organic solvents of such transaminase mutants are improved. The present application solves the problem of poor tolerance of extreme environments by transaminase in the prior art, and is suitable for the field of enzyme engineering.

## Description

### Technical Field

The present disclosure relates to the field of enzyme engineering, in particular to a transaminase mutant and an application thereof.

### Background

Chiral amines are important intermediates in the synthesis of many chiral drugs and are also main components of optically pure drugs containing amino groups. Nervous drugs, vascular drugs, antihypertensive drugs, anti-infective drugs, and vaccines all use the chiral amines as the intermediates. Transaminases use ketone compounds as raw materials and may efficiently produce the chiral amines by stereoselective transamination. However, there are many problems in applications of free enzyme enzymatic methods in large-scale production, such as low enzyme activity and high enzyme amount may cause increased fermentation costs; and it is prone to denaturation and deactivation due to the influence of organic solvents in reaction systems, and post-treatment recovery products have serious emulsification phenomenon, resulting in a large amount of three wastes. In addition, during the process of separating product amines, they may only be removed and discarded by denaturing and deactivating enzymes to form precipitates, and may not be reused.

The research on immobilized enzymes began in 1910 and was officially conducted in the 1960s. In the 1970s, it was widely developed worldwide. Immobilization of enzymes is a type of technologies that uses solid materials to bind or restrict enzymes to a certain area, so that they are still capable of performing their unique catalytic reactions, and may be recovered and reused. Compared with free enzymes, the immobilized enzymes not only maintain their high efficiency, specificity, and mild enzymatic catalytic reaction characteristics, but also overcome the deficiencies of the free enzymes, and present a series of advantages such as high storage stability, easy separation and recovery, reusable for many times, continuous and controllable operations, and simple process. The immobilized enzymes are not only highly active in the fields of chemistry, biology, bioengineering, medicine, and life sciences, but also are rapidly developed and widely applied. It meets the strategic requirements of sustainable development due to the ecological and environmental effects of saving resources and energy, reducing or preventing pollution. Therefore, the immobilized enzymes already become one of the main research contents in the field of modern enzyme engineering. The immobilized enzymes have a broad prospect in industrial production due to their relatively high stability.

The use of the immobilized enzymes for batch stirring reactions may achieve the reuse of the enzymes, and greatly alleviate the emulsification phenomenon in the post-treatment and reduce the generation of three wastes. However, the batch stirring reaction requires recovery operations of the immobilized enzymes, and intermittent feeding may affect production capacity. A shearing force generated by stirring may easily inactivate the enzymes, and the stirring power consumption is relatively large. An immobilized enzyme continuous flow enzymatic reaction involves filling the immobilized enzyme into a pipe-type reactor, using a pump to inject a substrate at an appropriate flow rate from an inlet of the reactor, and receiving a product at an outlet. The continuous flow reaction is simple in device, low in energy consumption, manpower-saving, high in production capacity, and more suitable for scale-up applications of the immobilized enzymes.

Since most of existing substrate ketones (amino receptors) are poor in water solubility, the continuous reaction may not be performed in a pure aqueous phase. In order to achieve the continuous reaction, the relatively high temperature is required, or sufficient organic co-solvents need to be added to dissolve the substrate. However, extreme temperatures, pH, and organic solvents and the like may easily inactivate the transaminase. Therefore, it is necessary to modify the transaminase, as to improve the problem of limited applications due to its poor stability in the extreme environments such as high temperatures and organic solvents.

In the early stage, the transaminase is modified to obtain a mutant (CN108384767B) that may withstand 45°C high temperature and 50% high concentration organic solvent, and the transaminase mutant is successfully immobilized by carrier-free immobilization cross-linked enzyme aggregate (CLEA), amino carrier covalent immobilization, and other technologies, the reuse frequency may reach 18 times. In order to achieve the continuous reaction in a packed bed reactor, a high-concentration co-solvent, namely ≥35% (v/v) methanol solution, is used to completely dissolve the substrate. Under this high-concentration organic solvent condition, the carrier immobilized enzyme successfully achieves the continuous transaminase enzymatic reaction, the retention time is 600 min, it is continuously operated for 240 h, and the conversion rate is not reduced. However, the retention time of 600 min is still relatively low in the industrial applications. In order to improve productivity, the activity and stability of the immobilized enzymes need to be further improved. Protein engineering technologies may directionally modify enzyme molecules from the perspective of the molecular structure to obtain mutants with improved performances (such as activity, tolerance, and substrate spectrum). The immobilization technologies of the enzymes may usually improve the storage and usage stability of the enzymes, as well as the tolerance to temperature, pH, organic solvents and the like. Most importantly, the immobilized enzymes may be recycled and reused, to reduce the enzyme waste. Usually, after the immobilization of the enzymes, especially the immobilization by a covalent binding mode, the enzyme activity may be decreased, namely the enzyme activity recovery is <100%. In order to improve the enzyme activity recovery of the immobilized enzymes, a most direct and effective method is to firstly modify the free enzyme directionally, as to improve the tolerance of the enzyme to more extreme conditions, reduce the activity loss of the enzyme in the covalent immobilization process, and further improve the service life of the immobilized enzyme.

The carrier immobilized enzyme is large in mass transfer resistance and low in reaction efficiency since the enzyme is immobilized in the porous structure of the carrier, and the contact between the enzyme and the substrate is limited in the reaction. The carrier-free immobilized enzyme, namely a cross-linked enzyme, may increase the contact area and efficiency between the enzyme and the substrate, and thereby the reaction efficiency is improved. If the cross-linked enzyme with the relatively low mass transfer resistance is applied to the continuous reaction, it may greatly improve the productivity. However, due to the lack of a solid carrier, the cross-linked enzyme is relatively weak in strength. If it is filled in pipelines, when reaction solution flows through, it may generate a significant column pressure, so that the continuous flow reaction may not be performed. Therefore, developing a cross-linked enzyme with high hardness and good mechanical strength is another direction to improve the productivity of the immobilized enzymes.

### Summary

A main purpose of the present disclosure is to provide a transaminase mutant and an application thereof, as to further improve tolerance of transaminases to extreme environments in existing technologies.

In order to achieve the above purpose, according to an aspect of the present disclosure, a transaminase mutant is provided, the transaminase mutant has an amino acid mutation based on a sequence shown in SEQ ID NO: 1, and the amino acid mutation is C418Q, C418W, W60Y, Y168A, V379W, V379L, V379M, W60Y+Y168A, W60Y+V379W, W60Y+V379L, W60Y+V379M, W60Y+C418Q, W60Y+C418W, Y168A+V379W, Y168A+V379L, Y168A+V379M, Y168A+C418Q, Y168A+C418W, V379W+C418Q, V379W+C418W, V379L+C418Q, V379L+C418W, V379M+C418Q, V379M+C418W, W60Y+Y168A+V379W, W60Y+Y168A+V379L, W60Y+Y168A+V379M, W60Y+Y168A+C418Q, W60Y+Y168A+C418W, W60Y+V379W+C418Q, W60Y+V379W+C418W, W60Y+V379L+C418Q, W60Y+V379L+C418W, W60Y+V379M+C418Q, W60Y+V379M+C418W, Y168A+V379W+C418Q, Y168A+V379W+C418W, Y168A+V379L+C418Q, Y168A+V379L+C418W, Y168A+V379M+C418Q, Y168A+V379M+C418W, W60Y+Y168A+V379W+C418Q, W60Y+Y168A+V379L+C418Q, W60Y+Y168A+V379M+C418Q, W60Y+Y168A+V379W+C418W, W60Y+Y168A+V379L+C418W, or W60Y+Y168A+V379M+C418W; alternatively, the amino acid sequence of the transaminase mutant has a mutation site in the mutated amino acid sequence, and has more than 85% of identity with the mutated amino acid sequence, and has the transaminase activity.

Further, the amino acid sequence of the transaminase mutant has more than 90%, preferably more than 95%, and more preferably more than 99% of the identity with the mutated amino acid sequence, and has the transaminase activity.

Further, the transaminase mutant is derived from *Chromobacterium violaceum.*

According to a second aspect of the present application, a DNA molecule is provided, and the DNA molecule encodes the above transaminase mutant.

According to a third aspect of the present application, a recombinant plasmid is provided, and the recombinant plasmid is linked to the above DNA molecule.

According to a fourth aspect of the present application, an immobilized transaminase is provided, and the immobilized transaminase comprises the above transaminase mutant.

Further, the immobilized transaminase comprises a cross-linked immobilized enzyme aggregate of the transaminase mutant; preferably, the cross-linked immobilized enzyme aggregate is a glutaraldehyde cross-linked immobilized enzyme aggregate; preferably, the cross-linked immobilized enzyme aggregate is an immobilized enzyme aggregate cross-linked with polyetherimide (PEI) activated by glutaraldehyde; and more preferably, the cross-linked immobilized enzyme aggregate is an immobilized enzyme aggregate cross-linked with cofactor-activated PEI by glutaraldehyde.

Further, the immobilized transaminase comprises an immobilized enzyme that combine the transaminase mutant with a carrier; preferably, the carrier is a cross-linked polymer resin sphere; preferably, the resin sphere comprises but not limited to a macroporous adsorption resin sphere, an amino resin sphere, or an epoxy resin sphere; preferably, a matrix of the macroporous adsorption resin sphere is functional group-free polystyrene; more preferably, the macroporous adsorption resin comprises but not limited to NKA9, LXEP120, AB-8, ECR8806, XAD-7 (Ambolet) or D3520; preferably, the amino resin sphere is a cross-linked polymer resin sphere with an amino functional group; more preferably, the functional group of the amino resin sphere is an amino functional group of a length C1-C4 or C5-C10; more preferably, the functional group of the amino resin sphere is an amino functional group with a carbon chain arm of a C2 or C6 length; more preferably, the amino resin sphere is a polymethacrylate resin sphere, a polystyrene resin sphere, or a methacrylate-styrene copolymer resin sphere; more preferably, the amino resin sphere comprises but not limited to Lifetech^{™}ECR8309, Lifetech^{™}ECR8409, Seplite^{®}LX1000HA, or Seplite^{®}LXEPHA; preferably, the epoxy resin sphere is a polymer resin sphere with an epoxy functional group; more preferably, the epoxy resin sphere has an epoxy functional group with a carbon chain arm of a C2-C8 length; more preferably, the epoxy resin sphere comprises but not limited to a polymethacrylate resin sphere, a polystyrene resin sphere or a methacrylate-styrene copolymer resin, having the epoxy functional group; and more preferably, the epoxy resin sphere comprises but not limited to Seplite^{®}LXHFA001 or Lifetech^{™}ECR8285.

Further, the immobilized transaminase is an affinity carrier-immobilized enzyme formed by affinity adsorption between the transaminase mutant and a metal affinity adsorption-type carrier;
comprises; preferably, the carrier is a resin sphere with a polymethacrylic acid matrix; more preferably, the resin sphere uses nitrilotriacetic acid (NTA) or iminodiacetic acid (IDA) as a ligand for chelating metal ions; more preferably, the resin sphere chelates the metal ion ligand at the terminal; further preferably, the metal ion ligand comprises but not limited to Ni²⁺, Co²⁺, Cu²⁺, or Fe³⁺; further preferably, the resin sphere comprises but not limited to IMAC-Ni or MIDA-Ni; and further preferably, the resin sphere comprises but not limited to Bio-Rad Profinity ^{™} IMAC Resin, Purolite Chromalite ^{™} MIDA/M/Ni, Purolite Chromalite ^{™} MIDA/M/Co, Purolite Chromalite ^{™} MIDA/M/Cu, or Purolite Chromalite ^{™} MIDA/M/Fe.

According to a fifth aspect of the present application, a method for producing a chiral amine with a transaminase is provided, which comprises a step of catalyzing a transamination reaction of a ketone compound and an amino donor with the transaminase, and the transaminase is the above transaminase mutant or immobilized transaminase.

Further, the method is a batch reaction or a continuous reaction; preferably, the batch reaction or the continuous reaction is performed in an aqueous phase or an organic phase; preferably, conditions for performing the reaction in the aqueous phase are that: an organic solvent as a co solvent, which is mutually soluble with water and accounts for not greater than 70% by volume of the total system, is added to an aqueous solution for catalytic reaction; further, the organic solvent is methanol, ethanol, or dimethyl sulfoxide; preferably, conditions for performing the reaction in the organic phase are that: a catalytic reaction is performed in a water-saturated organic solvent which is not mutually soluble with water, and further, the catalytic reaction is performed in a water-saturated methyl tert-butyl ether solution or water saturated isopropyl acetate solution; preferably, the immobilized transaminase is the above immobilized transaminase, and the immobilized transaminase achieves the packed bed continuous reaction by filling in a pipeline; preferably, the continuous use time of the immobilized enzyme is not less than 400 h; and preferably, the space-time yield of the immobilized enzyme is 3.6-7.2 mol/L/day.

Further, the above ketone compound is 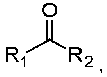 herein R₁ and R₂ are each independently a C1-C8 alkyl, a C5-C10 cycloalkyl, or a C6-C10 aryl, or R₁ and R₂ form a C5-C10 heterocyclic group or a C5-C10 carbocyclic group together with carbon on a carbonyl group, heteroatoms in the C5-C10 heterocyclic group are each independently selected from at least one of nitrogen, oxygen, and sulfur, and the aryl in the C6-C10 aryl, the carbocyclic group in the C5-C10 heterocyclic group or the heterocyclic group in the C5-C10 heterocyclic group are each independently unsubstituted or substituted by at least one group of halogen, alkoxy or alkyl; preferably, R₁ and R₂ are each independently a C5-C10 heterocyclic alkane, heteroatoms in the C5-C10 heterocyclic alkane are each independently selected from at least one of nitrogen, oxygen, and sulfur, and the carbocyclic group of the C5-C10 heterocyclic alkane is unsubstituted or substituted by at least one group of halogen, alkoxy or alkyl; preferably, the ketone compound may be 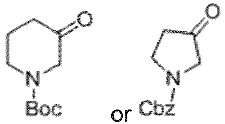 , herein a product of the transamination reaction is

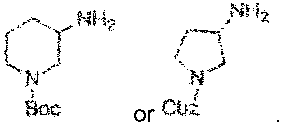

According to a sixth aspect of the present application, a production method for an immobilized transaminase is provided, and the production method comprises: a step of immobilizing a transaminase mutant, herein the transaminase mutant is the above transaminase mutant.

Further, the step of immobilizing the transaminase mutant in the production method comprises: the transaminase mutant is cross-linked and immobilized to form an immobilized enzyme aggregate; preferably, the step of cross-linking and immobilizing the transaminase mutant to form the immobilized enzyme aggregate comprises: crosslinking an enzyme precipitated by a precipitant with glutaraldehyde to form the immobilized enzyme aggregate; or crosslinking an enzyme precipitated by a precipitant with a glutaraldehyde-activated PEI to form the immobilized enzyme aggregate; or crosslinking the transaminase mutant with a cofactor-activated PEI via glutaraldehyde to form the immobilized enzyme aggregate. Further, the step of immobilizing the transaminase mutant in the production method comprises: the transaminase mutant is linked with a carrier to form the immobilized enzyme; preferably, the carrier is a cross-linked polymer resin sphere; preferably, the resin sphere comprises but not limited to a macroporous adsorption resin sphere, an amino resin sphere, or an epoxy resin sphere; preferably, a matrix of the macroporous adsorption resin sphere is functional group-free polystyrene; more preferably, the macroporous adsorption resin comprises but not limited to NKA9, LXEP120, AB-8, ECR8806, XAD-7 or D3520; preferably, the amino resin sphere is a cross-linked polymer resin sphere with an amino functional group; more preferably, the functional group of the amino resin sphere is an amino functional group with a carbon chain arm of a C2 or C6 length; more preferably, the amino resin sphere is a polymethacrylate resin sphere, a polystyrene resin sphere, or a methacrylate-styrene copolymer resin sphere; more preferably, the amino resin sphere comprises but not limited to Lifetech^{™}ECR8309, Lifetech^{™}ECR8409, Seplite^{®}LX1000HA, or Seplite^{®}LXEPHA; preferably, the epoxy resin sphere is a polymer resin sphere with an epoxy functional group; more preferably, the epoxy resin sphere has an epoxy functional group with a carbon chain arm of a C2-C8 length; more preferably, the epoxy resin sphere is a polymethacrylate resin sphere, a polystyrene resin sphere or a methacrylate-styrene copolymer resin, having the epoxy functional group; and more preferably, the epoxy resin sphere comprises but not limited to Seplite^{®}LXHFA001 or Lifetech^{™}ECR8285.

Further, the step of immobilizing the transaminase mutant in the production method comprises: the transaminase mutant is affinity-adsorbed with the carrier to form an affinity immobilized enzyme by affinity adsorption; preferably, the transaminase mutant is co-incubated with the carrier, to obtain the affinity immobilized enzyme; preferably, the transaminase mutant has a plurality of His-tags; preferably, the carrier is a resin sphere with a polymethacrylic acid matrix; more preferably, the resin sphere contains a NTA or IDA ligand; more preferably, the resin sphere chelates the metal ion ligand at the terminal; further preferably, the metal ion ligand comprises but not limited to Ni²⁺, Co²⁺, Cu²⁺, or Fe³⁺; further preferably, the carrier comprises but not limited to IMAC-Ni or MIDA-Ni; and further preferably, the carrier comprises but not limited to Bio-Rad Profinity^{™}IMAC Resin, Purolite Chromalite^{™}MIDA/M/Ni, Purolite Chromalite^{™}MIDA/M/Co, Purolite Chromalite^{™}MIDA/M/Cu, or Purolite Chromalite^{™}MIDA/M/Fe.

By applying technical schemes of the present disclosure, the mutant with the improved activity, stability, and tolerance to temperature, pH, and organic solvent is obtained by directed evolution screening, not only the usage amount of the enzyme in the production applications is reduced, but also the possibility of preparing various immobilized enzymes is greatly improved. By immobilizing the above transaminase after the directed evolution (self-crosslinking or covalent-binding with the carrier), the applications of the immobilized transaminase in the aqueous phase reaction and the organic phase reaction may be achieved, so that the enzyme is easily separated from the reaction system and the emulsification phenomenon caused by the residual enzyme protein during the reaction post-treatment process is reduced. At the same time, the immobilized transaminase mutant may withstand various extreme environments with low activity loss, high reuse times, and high space-time yield, and is suitable for the industrial continuous transamination reaction.

### Detailed Description of the Embodiments

It should be noted that in the case without conflicting, embodiments in the present application and features in the embodiments may be combined with each other. The present disclosure is described in detail below in combination with the embodiments.

Term explanation:
Immobilized enzyme: refers to an enzyme of which catalytic activity may be repeatedly and continuously used within a certain spatial range. Usually, enzyme catalyzed reactions are all performed in aqueous solution, while the immobilized enzyme is that a water-soluble enzyme is treated with a physical or chemical method, so that it is insoluble in water but still has the enzymatic activity. After enzyme immobilization, the stability is generally increased, it is easy to separate from the reaction system, is easy to control, may be used for many times, is convenient for transportation and storage, and is beneficial to automated production, but the activity is decreased and the usage range is reduced.

Site-directed mutagenesis (or site-specific mutagenesis) refers to a method of introducing a specific base pair in a designated site of a target DNA fragment. By changing the nucleotide sequence in the gene specific site, the amino acid sequence encoded is changed, it is commonly used to research the effects of (a) certain amino acid residues on the protein structure and function. In the rational design of the enzymes, the site-specific mutagenesis method may be used to screen mutated enzymes with improved catalytic activity, substrate specificity, and/or stability.

Saturation mutation is a method of acquiring a mutant with a target amino acid substituted by 19 other amino acids in a short period of time by modifying a coding gene of a target protein. This method is not only a powerful tool for protein directed modification, but also an important means for researching a protein structure-function relationship. The saturation mutation often obtains a more ideal evolutionary body than the single point mutation. In addition, for these problems that may not be solved by the site-specific mutagenesis method, it is precisely the unique advantage of the saturation mutation method.

Activity recovery refers to the ratio of the total activity of the immobilized enzyme to the total activity of the enzyme used for immobilization. When the enzymes are immobilized, there are always some enzymes that are not bound to the carrier. Measuring the enzyme activity recovery may determine the effect of immobilization, and in general, the activity recovery is less than 1.

Space-time yield (STY) refers to an amount of a target product (or an amount of a raw material fed) obtained per unit volume or unit area of a device within a unit time.

Technical schemes and technical effects of the present application are described below in combination with experiments.

### I. Screening for mutants with improved enzyme activity and tolerance to temperature, pH, and organic reagent

This scheme used TA-Cv-1 (R416T+T7C+S47C+R405E+K90G+A95P+K304D+Q380L+1297L, CN108384767B, and the sequence as shown in SEQ ID NO: 1) as a template. By a method of directed evolution, the activity, and temperature and organic solvent tolerance of TA-Cv-1 were further improved, so that it was applied for the catalytic reaction better after immobilization.

The sequence of SEQ ID NO: 1 was as follows:

MQKQRTCSQWRELDAAHHLHPFTDTASLNQAGARVMTRGEGVYLWDCEGNKIIDGMAGLW
CVNVGYGRKDFAEAARRQMEELPFYNTFFGTTHPPVVELSSLLAEVTPAGFDRVFYTNSGSESVD
TMIRMVRRYWDVQGKPEKKTLIGRWNGYHGSTIGGASLGGMKYMHEQGDLPIPGMAHIEQPWW
YKHGKDMTPDEFGVVAARWLEEKILEIGADKVAAFVGEPIQGAGGVIVPPATYWPEIERICRKYDVL
LVADEVICGFGRTGEWFGHQHFGFQPDLFTAAKGLSSGYLPLGAVFVGDRVAEGLIAGGDFNHGF
TYSGHPVCAAVAHANVAALRDEGIVQRVKDDIGPYMQKRWRETFSRFEHVDDVRGVGMVLAFTL
VKNKAKRELFPDFGEIGTLCEDIFFRNNLIMTACGDHIVSAPPLVMTRAEVDEMLAVAERCLEEFEQ
TLKARGLA.

TA-Cv-1 was used as a template, a site-specific mutation primer was designed to perform a site-specific mutation (F22M, M56A, M56G, M56G, W60Y, F89V, Y168A, I262L, V423L, V423I, V379W, C418W) in 8 sites. After protein structure analysis, the sites V379 and C418 were key sites for improving the enzyme stability, the saturation mutation was mainly performed on these two sites, and the primer sequences of the saturation mutation was shown in Table 1 below.

**Table 1: Saturation mutation primer**

| | Saturation mutation | Sequence |
|---|---|---|
| 1 | TA-CV-1-V379NDT-R | agcgtgaacgccagAHNcataccaacaccgcg (SEQ ID NO: 2) |
| | TA-CV-1-V379NDT-F | cgcggtgttggtatgNDTctggcgttcacgct (SEQ ID NO: 3) |
| 2 | TA-CV-1-V379VHG-R | agcgtgaacgccagCDBcataccaacaccgcg (SEQ ID NO: 4) |
| | TA-CV-1-V379VHG-F | cgcggtgttggtatgVHGctggcgttcacgct (SEQ ID NO: 5) |
| 3 | TA-CV-1-C418NDT-R | cacgatgtgatcgccAHNggcagtcataatc (SEQ ID NO: 6) |
| | TA-CV-1-C418NDT-F | gattatgactgccNDTggcgatcacatcgtg (SEQ ID NO: 7) |
| 4 | TA-CV-1-C418VHG-R | cacgatgtgatcgccCDBggcagtcataatc (SEQ ID NO: 8) |
| | TA-CV-1-C418VHG-F | gattatgactgccVHGggcgatcacatcgtg (SEQ ID NO: 9) |

A complete linear fragment was obtained by a full plasmid polymerase chain reaction (PCR), and the above PCR product was digested by Dpnl to remove a parental template of a starting gene, transformed into *Escherichia coli* BL21 (DE3), spread in an LB culture dish containing 50 µg/mL ampicillin, and cultured overnight at 37°C. The site-specific mutagenesis used gene sequencing to determine the mutation site. The saturation mutations were screened by high-throughput screening and then gene-sequenced to determine the mutation site. The site-specific mutagenesis means was used to obtain a mutant plasmid with a target gene with pET-22b(+) as an expression vector. The mutant plasmid was transformed into *Escherichia coli* cells, and the optimal conditions for the transaminase induced expression were as follows: 25°C, and 0.1 mM isopropyl-β-d-thiogalactoside (IPTG) induction overnight. A crude enzyme was obtained by a method of ultrasonic cell-breaking.

After enzyme solution expressed by a mutant strain was treated in an extreme environment of 50-60°C, pH 10, and 50%~70% dimethylsulfoxide (DMSO) or 40% MeOH for 1 h, Substrate 1 (N-Boc-3-piperidone) or Substrate 2 (N-Cbz-3-pyrrolidone) was added, the conversion rate was detected after it was continuously reacted under these conditions for 16 h, and the mutant with the improved temperature, pH, and organic solvent tolerance was screened in this way. The mutant with the mutation sites at W60Y, Y168A, V379W, V379L, V379M, C418Q, and C418W showed a 1.05-1.2 fold increase in tolerance to 50°C, pH 10, and 50% DMSO compared to the wild-type parent. The tolerance to 60°C, pH 10, and 60% DMSO environment was 1.3-2.5 times higher than that of the wild-type parent, the tolerance to 60°C, pH 10, 70% DMSO environment was 3.3-4.2 times higher than that of the wild-type parent, and the tolerance to 60°C, pH 10, 40% MeOH environment was 2.7-4 times higher than that of the wild-type parent.

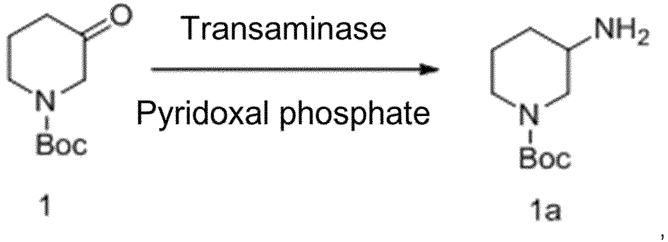

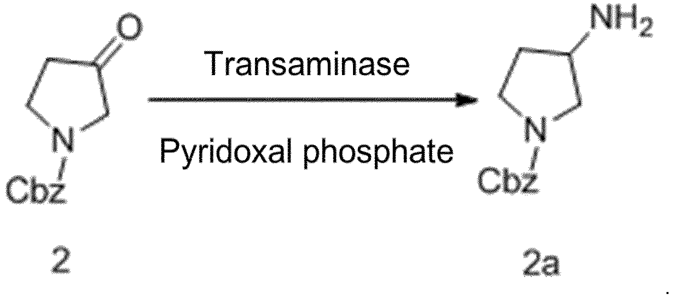

In order to further evolve the enzyme with better stability and tolerance, this research performed a multi-point combination mutation on these sites that had a positive effect on transaminase stability and tolerance, to obtain a multi-point mutant with the further improved stability and tolerance by a method of directional screening. Examples of the mutation site for the combined mutation were as follows: W30Y+Y168A, W60Y+V379W, W60Y+V379L, W60Y+V379M, W60Y+C418Q, W60Y+C418W, Y168A+V379W, Y168A+V379L, Y168A+V379M, Y168A+C418Q, Y168A+C418W, V379W+C418Q, V379W+C418W, V379L+C418Q, V379L+C418W, V379M+C418Q, V379M+C418W, W60Y+Y168A+V379W, W60Y+Y168A+V379L, W60Y+Y168A+V379M, W60Y+Y168A+C418Q, W60Y+Y168A+C418W, W60Y+V379W+C418Q, W60Y+V379W+C418W, W60Y+V379L+C418Q, W60Y+V379L+C418W, W60Y+V379M+C418Q, W60Y+V379M+C418W, Y168A+V379W+C418Q, Y168A+V379W+C418W, Y168A+V379L+C418Q, Y168A+V379L+C418W, Y168A+V379M+C418Q, Y168A+V379M+C418W, W60Y+Y168A+V379W+C418Q, W60Y+Y168A+V379L+C418Q, W60Y+Y168A+V379M+C418Q, W60Y+Y168A+V379W+C418W, W60Y+Y168A+V379L+C418W, or W60Y+Y168A+V379M+C418 W.

The mutant of the present application might also be a mutant with the above mutation site and more than 85% (preferably more than 90%, or more than 95%, or more than 99%, or more than 99.95%, or even more than 99.99%) of identity with the mutated amino acid sequence, and has the transaminase activity. More preferably, these mutants are all derived from *Chromobacterium violaceum* and have the transaminase activity.

The mutant plasmid was transformed into *Escherichia coli* cells, and the optimal conditions for the transaminase induced expression were as follows: 25°C, and 0.1 mM IPTG induction overnight. A crude enzyme was obtained by the method of ultrasonic cell-breaking.

Under more extreme conditions, such as 70°C, pH 10, and environment containing 45% MeOH, the enzyme solution was treated for 1 h, Substrate 1 was added, it was continuously reacted under these conditions for 16 h, and the conversion rate was detected. After treatment under these conditions, the activity of the combined mutation was increased by 2-6 times compared to the wild-type parent.

### II. Preparation of transaminase cross-linked enzyme aggregates (CLEAs)

The transaminase immobilized by the cross-linking method was selected from the wild-type parent (TA-Cv-1, as well as the mutants C418Q+V379L and C418Q+V379M obtained on the basis of the wild-type parent), buffer solution for preparing the enzyme solution contained 0.4-1 mg/mL of pyridoxal phosphate (PLP), and enzyme solution pH was 7.0-8.0. A precipitant used for preparing an enzyme protein precipitate was ethanol, ammonium sulfate or acetonitrile. A cross-linked aggregate for preparing the enzyme protein was prepared with a cross-linking agent of 25% glutaraldehyde solution or glutaraldehyde treated with polyethylene imine (CN111235140B), and the final concentration of glutaraldehyde was 100 mM-500 mM. The cross-linked enzyme aggregate prepared might be directly catalyzed and reacted by the aqueous cross-linked enzyme filtered in an aqueous phase, or the aqueous cross-linked enzyme was freeze-dried to obtain dry powder before application, and the freeze-dried powder might also be used for a reaction in an organic solvent phase.

The activity recovery of the cross-linked enzyme aggregate might reach more than 80%. After the cross-linked enzyme aggregate was used once, it might be recycled by methods such as centrifugation or filtration before reuse. The number of repeated uses might be counted within the range of activity loss <5%. The cross-linked enzyme aggregate of the mutant might be reused for 9-30 times in the aqueous phase reaction, and the activity loss was <5%. It was reacted in 100% organic phase solvent and might be reused for 19-35 times.

The transaminase cross-linked enzyme aggregate had the improved activity, stability, and mechanical strength compared to the prior art, and might be used for the packed bed continuous reaction, especially for carrier-free immobilization.

### III. Immobilization method and activity of transaminase

### 1. Adsorption immobilization method for transaminase

The resin carrier used in the adsorption immobilization method of this research was a macroporous adsorption resin carrier. Table 2 listed carrier information used for the adsorption immobilization method in this research.

**Table 2: Name, matrix, functional group, and type of adsorption immobilization carrier**

| Carrier name | Matrix | Functional group | Type |
|---|---|---|---|
| NKA9 | Polystyrene | Free | Macroporous adsorption resin |
| XAD-7 | Polystyrene | Free | Macroporous adsorption resin |
| AB-8 | Polystyrene | Free | Macroporous adsorption resin |
| D3520 | Polystyrene | Free | Macroporous adsorption resin |
| LXEP-120 | Polystyrene | Free | Macroporous adsorption resin |
| ECR8806 | Polystyrene | Free | Macroporous adsorption resin |

The transaminase immobilized by the covalent binding method in this research was selected from mutants (W60Y, C418Q, C418Q+V379L, C418Q+V379M) for the adsorption immobilization. The enzyme solution was directly mixed with the carrier, it was stirred overnight at 20°C and a low speed of 80 rpm, and filtered to collect a precipitate, and the precipitate was washed with buffer solution.

### 2. Covalent immobilization method for transaminase

The resin carrier used in the covalent immobilization method of this research included an amino-type carrier and an epoxy-type carrier. Table 3 listed carrier information used for the covalent immobilization method in this research.

**Table 3: Name, matrix, functional group, and type of covalent immobilization carrier**

| Carrier name | Matrix | Functional group | Type |
|---|---|---|---|
| ECR8309 | Polymethacrylate | Short chain amino (C2) | Amino-type carrier |
| ECR8409 | Polymethacrylate | Long chain amino (C6) | Amino-type carrier |
| ECR8285 | Polymethacrylate | Epoxy | Epoxy-type carrier |
| LX1000HA | Polymethacrylate | Long chain amino (C6) | Amino-type carrier |
| LX1000HFA | Polymethacrylate | Amino epoxy | Epoxy-type carrier |

The above amino-type carrier included but not limited to Lifetech^{™}ECR8309 and Lifetech^{™}ECR8409 from Purolite, and Seplite^{®}LX1000HA or Seplite^{®}LXEPHA from SunResin Technology; and the epoxy-type carrier included but not limited to Seplite^{®}LXHFA001 from SunResin Technology or Lifetech^{™}ECR8285 from Purolite.

This transaminase in the present research covalently combined directly with a resin having the epoxy functional group, and covalently combined with a glutaraldehyde-activated resin having a short chain or a long chain amino functional group.

The transaminase immobilized by the covalent binding method in this research was selected from the wild-type parent (TA-Cv-1) and mutants (W60Y, C418Q, C418Q+V379L, C418Q+V379M), buffer solution used to prepare the enzyme solution contained 0.4-1 mg/mL of PLP, buffer solution pH was 7.0-8.0, and the buffer solution was PB, Na₂HPO₄-NaH₂PO₄, Tris-Cl, or boric acid-sodium hydroxide buffer.

Method of covalent immobilization to the amino-type carrier: firstly, the carrier was activated with glutaraldehyde, and then the enzyme solution was added; it was incubated overnight at 20°C, and filtered to collect a precipitate, and the precipitate was washed with the buffer solution.

Method of covalent immobilization to the epoxy-type carrier: the enzyme solution was directly mixed with the carrier; it was incubated overnight at 20°C, and then placed stilly for 20 h, and filtered to collect a precipitate, and the precipitate was washed with the buffer solution.

### 3. Affinity immobilization method for transaminase

The carrier used in the affinity immobilization method of this research was a resin modified by a polymer film and chelated with metal ions at the terminal. The chelated metal ion included but not limited to Ni²⁺, Co²⁺, or Fe³⁺. Table 4 listed carrier information used for the affinity immobilization method in this research.

**Table 4: Name of affinity immobilization carrier and chelated metal ion**

| Carrier name | Chelated metal ion |
|---|---|
| IMAC-Ni | Ni²⁺ |
| MIDA-Ni | Ni²⁺ |

The above IMAC-Ni carrier included but not limited to Profinity^{™}IMAC Resin (https://ww w.bio-rad.com/zh-cn/product/profinity-imac-resin? ID=e54ab03c-d281-4e6b-aa0d-193b7737626d) from Bio-Rad; and the MIDA-Ni carrier included but not limited to Chromalite^{™}MIDA/M/Ni, MIDA/M/Co, MIDA/M/Cu, and MIDA/M/Fe (https://www.purolite.com/ls-product/spectrachrom-kit) from Purolite.

The transaminase immobilized by the affinity binding method in this research was selected from the wild-type parent (TA-Cv-1), and mutants (W60Y, C418Q, C418Q+V379L, C418Q+V379M). 5 mL of sodium dihydrogen phosphate-disodium hydrogen phosphate buffer solution containing 0.4 mg/mL of PLP (50 mM, pH 7.0) was added to the carrier, and 0.1 g of the enzyme was simultaneously added, it was stirred at 20°C and a low speed of 80 rpm for 40-60 min. A supernatant was removed, and a precipitate was washed with the buffer solution for 3-4 times.

The immobilized enzyme prepared might be dried by nitrogen-blowing, vacuum-drying, freeze-drying, and other methods.

### 4. Activity of immobilized transaminase

In this research, the transaminase was immobilized onto the above carrier by a mode of covalent binding or affinity adsorption. It was immobilized onto the short chain amino-type carrier, and the activity recovery was 50% -95%; it was immobilized onto the long chain amino-type carrier, and the activity recovery was 90%-100%; it was immobilized onto the epoxy-type carrier, and the activity recovery was 80% -100%; it was immobilized onto the affinity carrier, and the activity recovery was 90%-100%; and it was immobilized onto the adsorption carrier, the activity was detected in the organic phase reaction, and the activity recovery was >100%.

The immobilized enzyme might be recycled by methods such as filtration or liquid suction of an injector, and might be reused. The number of repeated uses might be counted within the range of activity loss <5%. It was immobilized onto the short chain amino-type carrier, some enzymes might be reused for 18 times; it was immobilized onto the long chain amino-type carrier, for certain mutant enzymes, the number of repeated uses might reach 25 times; it was immobilized onto the epoxy-type carrier, some enzymes might be reused for 20 times; it was immobilized onto the affinity carrier, it might be reused for 7-20 times, and might play a catalytic role in 100% organic solvent and was reused for 10-20 times, and the activity loss was <5%; and it was immobilized onto the adsorption carrier, and reacted in 100% organic solvent, it might be reused for 9-20 times.

The beneficial effects of the present application are further described below in combination with specific embodiments.

### Embodiment 1: Mutant 50°C, pH 10, 50% DMSO tolerance detection

A crude enzyme was treated at 50°C, pH 10, and DMSO concentration of 50% for 1 h, then, 0.1 g of Substrate 2 was added to a 10 mL reaction flask, and 4 eq of isopropylamine hydrochloride and 0.6-1 mg of PLP (5'-pyridoxal phosphate) were added. Then, 4 mg of the enzyme after the above treatment was added, and stirred at a constant temperature for 16 h at 50°C, pH 10, and DMSO concentration of 50%. The conversion rate of the system was detected by HPLC, and mutant reaction data was shown in Table 5 below.

**Table 5:**

| Mutation site | Conversion rate (%) |
|---|---|
| Wild-type parent(TA-Cv-1) | 79.6 |
| W60Y | 95.4 |
| Y168A | 83.8 |
| V379W | 88.9 |
| V379L | 91.7 |
| V379M | 91.1 |
| C418Q | 94.4 |
| C418W | 87.5 |

### Embodiment 2: Mutant 60°C, pH 10, 60% DMSO tolerance detection

A crude enzyme was treated at 60°C, pH 10, and DMSO concentration of 60% for 1 h, then, 0.1 g of Substrate 2 was added to a 10 mL reaction flask, and 4 eq of isopropylamine hydrochloride and 0.6 mg of PLP (5'-pyridoxal phosphate) were added. Then, 5 mg of the enzyme after the above treatment was added, and continuously stirred at a constant temperature for 16 h at 60°C, pH 10, and DMSO concentration of 60%. The conversion rate of the system was detected by HPLC, and mutant reaction data was shown in Table 6 below.

**Table 6:**

| Mutation site | Conversion rate (%) |
|---|---|
| Wild-type parent (TA-Cv-1) | 34.4 |
| W60Y | 84.3 |
| Y168A | 44.6 |
| V379W | 85.8 |
| V379L | 77.9 |
| V379M | 83.1 |
| C418Q | 69.0 |
| C418W | 75.2 |

### Embodiment 3: Mutant 60°C, pH 10, 70% DMSO tolerance detection

A crude enzyme was treated at 60°C, pH 10, and DMSO concentration of 70% for 1 h, then, 0.1 g of Substrate 2 was added to a 10 mL reaction flask, and 4 eq of isopropylamine hydrochloride and 0.6-1 mg of PLP (5'-pyridoxal phosphate) were added. Then, 10 mg of the enzyme after the above treatment was added, and continuously stirred at a constant temperature for 16 h at 60°C, pH 10, and DMSO concentration of 70%. The conversion rate of the system was detected by HPLC, and mutant reaction data was shown in Table 7 below.

**Table 7:**

| Mutation site | Conversion rate (%) |
|---|---|
| Wild-type parent (TA-Cv-1) | 21.9 |
| W60Y | 91.8 |
| Y168A | 90.2 |
| V379W | 72.3 |
| V379L | 74.9 |
| V379M | 83.4 |
| C418Q | 88.2 |
| C418W | 89.7 |

### Embodiment 4: Mutant 60°C, pH 10, 40% MeOH tolerance detection

A crude enzyme was treated at 60°C, pH 10, and MeOH concentration of 40% for 1 h, then, 0.1 g of Substrate 1 was added to a 10 mL reaction flask, and 4 eq of isopropylamine hydrochloride and 0.6-1 mg of PLP (5'-pyridoxal phosphate) were added. Then, 10 mg of the enzyme after the above treatment was added, and continuously stirred at a constant temperature for 16 h at 60°C, pH 10, and MeOH concentration of 40%. The conversion rate of the system was detected by HPLC, and mutant reaction data was shown in Table 8 below.

**Table 8:**

| Mutation site | Conversion rate (%) |
|---|---|
| Wild-type parent (TA-Cv-1) | 18.7 |
| W60Y | 59.9 |
| Y168A | 65.4 |
| V379W | 50.5 |
| V379L | 74.2 |
| V379M | 61.3 |
| C418Q | 74.4 |
| C418W | 71.1 |

### Embodiment 5: Mutant 70°C, pH 10, 45% MeOH tolerance detection

A crude enzyme was treated at 70°C, pH 10, and MeOH concentration of 45% for 1 h, then, 0.1 g of Substrate 1 was added to a 10 mL reaction flask, and 4 eq of isopropylamine hydrochloride and 0.6-1 mg of PLP (5'-pyridoxal phosphate) were added. Then, 10 mg of the enzyme after the above treatment was added, and continuously stirred at a constant temperature for 16 h at 70°C, pH 10, and MeOH concentration of 45%. The conversion rate of the system was detected by HPLC, and mutant reaction data was shown in Table 9 below.

**Table 9:**

| Mutation site | Conversion rate (%) |
|---|---|
| Wild-type parent (TA-Cv-1) | 8.7 |
| W60Y | 26.9 |
| Y168A | 18.1 |
| V379W | 29.4 |
| V379L | 39.6 |
| V379M | 48.2 |
| C418Q | 43.3 |
| C418W | 34.7 |
| C418Q+V379L | 50.9 |
| C418Q+V379M | 52.2 |

### Embodiment 6: Cross-linking immobilization

0.1 g of enzyme powder was dissolved in 2 mL phosphate buffer (0.1M PB, pH 7.0-8.0, containing 0.4-1 mg/mL of PLP (5'-pyridoxal phosphate)), 10 mL of ethanol, 7 mL of acetonitrile, PEG6000 solid (20-60% of the final concentration) or ammonium sulfate (40-70% of the final concentration) was slowly added as a precipitant under stirring in an ice-water bath. After being added, it was stirred for 40-60 min, and then 25% glutaraldehyde solution (100-500 mM of the final concentration) was added, it was stirred in the ice-water bath for 30-40 min before centrifugation or filtration, and after a precipitate was washed with the phosphate buffer for 3 times, it was stored at 4°C, and the precipitate might be directly applied to an aqueous phase reaction. Alternatively, a cross-linked enzyme aggregate might be freeze-dried, and freeze-dried cross-linked enzyme aggregate powder obtained might be applied in aqueous phase and organic phase reactions.

### Embodiment 7: Preparation of glutaraldehyde-activated PEI cross-linked immobilized enzyme aggregate (GA: glutaraldehyde, PEI: polystyrene imine, GP: GA activated PEI, or glutaraldehyde-activated PEI)

Preparation of activated PEI solution (GP): 2 g of glutaraldehyde and 4 g of PEI (3-70 KDa) were added to 100 mL of aqueous solution at pH 8.0, and it was stirred for 3 h.

Immobilization: 10 mL of an enzyme (the enzyme protein content was 100-200 mg/mL, correspondingly containing 1-5 mg/mL of a cofactor) was placed in a round bottom shake flask, and after it was cooled to 5-10°C, it was mixed for 10 min. A precipitant, such as ammonium sulfate (40%-70%), ethanol (50%-90%), or PEG (20%-60%), was slowly added, and mixed for 30-60 min, to obtain a precipitate enzyme. 15 mL of activated PEI solution was dropwise added to the precipitate enzyme, it was mixed for 10-30 min, and stilly placed for 1-2 h before centrifuge. An insoluble precipitate was collected, and passed through a 30-mesh sieve. It was washed with 0.1 M PB (pH 7.5).

### Embodiment 8: Preparation of cofactor-activated PEI cross-linked immobilized enzyme aggregate

Preparation of PEI solution activated by cofactor: PEI (Mw=3-70 KDa) was diluted with water until the final concentration of PEI was 1 g/mL-2 g/mL, and the pH value was 7.0-11.0. 1-5 mg/mL of the cofactor PLP was added to the PEI solution according to enzyme needs, and mixed at a room temperature for 30-60 min.

Immobilization: at 4-10°C, 7.5 mL of cofactor-activated PEl solution was dropwise added to 5 mL of enzyme solution (the enzyme protein content was 30-80 mg/mL, correspondingly containing the cofactor), and after being mixed for 30-60 min, cross-linking agent glutaraldehyde or PEG modified glutaraldehyde was added, and the final concentration of glutaraldehyde was 0.2-1 g/mL. After being mixed for 30-60 min, it was centrifuged to collect an insoluble precipitate, and then passed through a 30-mesh sieve. It was washed with 0.1 M PB (pH 7.0-8.0).

### Embodiment 9: Activity detection of cross-linked enzyme aggregate in aqueous phase reaction

In a 10 mL reaction flask, 4 eq of isopropylamine hydrochloride and 0.5 mg of PLP (5'- pyridoxal phosphate) were added, 0.1 M PB 7.0 was supplemented until the final volume of reaction solution was 1 mL, and then 3 mg of an enzyme or a cross-linked enzyme aggregate wet enzyme or cross-linked enzyme aggregate prepared from 3 mg of the enzyme was added, and it was stirred at 45°C for 16 h. The conversion rate of the system was detected by HPLC, and reaction data was shown in Table 10 below.

**Table 10:**

| Strain number | Immobilization form | Conversion rate (%) | Number of repeated uses with activity loss <5% |
|---|---|---|---|
| Wild-type parent | None | 64.2% | 1 |
| | Cross-linked enzyme | 52.8% | 8 |
| | Immobilized enzyme aggregate cross-linked by glutaraldehyde-activated PEI | 59.1 | 11 |
| | Immobilized enzyme aggregate cross-linked by cofactor-activated PEI | 46.8 | 6 |
| W60Y | None | 79.6 | 1 |
| | Cross-linked enzyme | 66.3 | 9 |
| | Immobilized enzyme aggregate cross-linked by glutaraldehyde-activated PEI | 74.2 | 12 |
| | Immobilized enzyme aggregate cross-linked by cofactor-activated PEI | 59.7 | 8 |
| V379L | None | 68.3% | 1 |
| | Immobilized enzyme aggregate cross-linked by glutaraldehyde-activated PEI | 62.9 | 10 |
| C418Q | None | 78.6% | 1 |
| | Cross-linked enzyme | 69.9 | 11 |
| | Immobilized enzyme aggregate cross-linked by glutaraldehyde-activated PEI | 64.6 | 12 |
| | Immobilized enzyme aggregate cross-linked by cofactor-activated PEI | 65.2 | 9 |
| C418Q+V379L | None | 88.6 | 1 |
| | Cross-linked enzyme | 83.8 | 19 |
| | Immobilized enzyme aggregate cross-linked by glutaraldehyde-activated PEI | 82.4 | 25 |
| | Immobilized enzyme aggregate cross-linked by cofactor-activated PEI | 74.5 | 22 |
| C418Q+V379M | None | 86.9 | 1 |
| | Cross-linked enzyme | 87.1 | 26 |
| | Immobilized enzyme aggregate cross-linked by glutaraldehyde-activated PEI | 85.7 | 30 |

### Embodiment 10: Activity detection of cross-linked enzyme in organic phase reaction

In a 10 mL reaction flask, 1 mL of water saturated methyl tert-butyl ether was added, then 100 mg of Substrate 1 and 4 eq of isopropylamine were added, and cross-linked enzyme aggregate freeze-dried powder prepared from 100 mg of enzyme powder was added. It was stirred at 30°C for 16 h. The conversion rate of the system was detected by HPLC, and reaction data was shown in Table 11 below.

**Table 11:**

| Strain number | Immobilization form | Conversion rate (%) | Number of repeated uses with activity loss <5% |
|---|---|---|---|
| W60Y | None | >92 | 8 |
| | Cross-linked enzyme freeze-dried powder | >92 | 19 |
| C418Q | None | >92 | 8 |
| | Cross-linked enzyme freeze-dried powder | >92 | 27 |
| C418Q+V379L | None | >92 | 11 |
| | Cross-linked enzyme freeze-dried powder | >92 | 32 |
| C418Q+V379M | None | >92 | 14 |
| | Cross-linked enzyme freeze-dried powder | >92 | 35 |

### Embodiment 11: Transaminase adsorption immobilization method

4 mL of PB containing 0.4 mg/mLof PLP (100 mM, pH 7.0) was added to a carrier, and 0.1 g of an enzyme wad added simultaneously. It was stirred overnight at 20°C and a low speed of 80 rpm. A supernatant was removed, and a precipitate was washed with the buffer solution for 3-4 times. The supernatant was removed, and the precipitate was dried by a nitrogen-blowing or freeze-drying method, and stored at 4°C.

### Embodiment 12: Activity detection of transaminase adsorption immobilized enzyme in organic phase reaction

In a 10 mL reaction flask, 1 mL of water saturated methyl tert-butyl ether was added, then 100 mg of Substrate 1 and 4 eq of isopropylamine were added, and an immobilized transaminase prepared from 50 mg of an enzyme was added. It was stirred at 30°C for 16 h. The conversion rate of the system was detected by HPLC, and reaction data was shown in Table 12 below.

**Table 12:**

| Strain number | Adsorption immobilization carrier | Conversion rate (%) | Number of repeated uses with activity loss <5% |
|---|---|---|---|
| C418Q | None | 68 | 1 |
| | ECR8806 | >92 | 13 |
| | XAD-7 | >92 | 9 |
| | AB8 | >92 | 10 |
| | N KA9 | >92 | 14 |
| | LXEP120 | >92 | 13 |
| W60Y | None | 65 | 1 |
| | ECR8806 | >92 | 11 |
| | AB8 | >92 | 10 |
| | D3520 | >92 | 10 |
| | LXEP120 | >92 | 11 |
| C418Q+V379L | None | 77 | 1 |
| | XAD-7 | >92 | 20 |
| | D3520 | >92 | 13 |
| | AB8 | >92 | 15 |
| | N KA9 | >92 | 17 |
| | LXEP120 | >92 | 19 |
| C418Q+V379M | None | 73 | 1 |
| | XAD-7 | >92 | 14 |
| | AB8 | >92 | 13 |
| | N KA9 | >92 | 17 |
| | LXEP120 | >92 | 20 |
| | ECR8806 | >92 | 20 |

Compared with the cross-linked enzyme and the carrier covalent immobilized enzyme, the adsorption immobilization enzyme had the high activity recovery, and higher activity and stability in the organic phase reaction.

### Embodiment 13: Amino-type carrier immobilized transaminase

Activated carrier: 1 g of a carrier was washed for 1-2 times with 20 mM low ion strength buffer, a supernatant was removed, and 4 mL of 2% glutaraldehyde (prepared by diluting 25% glutaraldehyde in a reagent grade with 20 mM low ion strength buffer) was added. It was activated at 20°C and 80 rpm for 1 h, and washed for 1-2 times with 20 mM buffer, and a supernatant was removed.

Immobilization: 4 mL of PB containing 0.4 mg/mL of PLP (20 mM, pH 7.0) was added to the activated carrier, and 0.1-0.2 g of an enzyme was added simultaneously. It was stirred overnight at 20°C and a low speed of 80 rpm. A supernatant was removed, and a precipitate was washed with the buffer solution for 3-4 times. The supernatant was removed, and the precipitate was dried by a nitrogen-blowing or freeze-drying method, and stored at 4°C.

### Embodiment 14: Epoxy-type carrier immobilized transaminase

1 g of a carrier was washed for 1-2 times with 100 mM PB, a supernatant was removed, 4 mL of Buffer (100 mM PB, pH 7.0, containing 1 M NaCl) was added, and 0.1-0.2 g of an enzyme was added simultaneously. It was stirred overnight (18-20 h) at 20°C and a low speed of 80 rpm, and placed stilly at 4°C for 20 h. The supernatant was removed, and a precipitate was washed with Buffer for 3-4 times, blowing-dried with nitrogen, and stored at 4°C.

### Embodiment 15: Activity detection of covalent immobilized transaminase in aqueous phase reaction

In a 10 mL reaction flask, 0.5 mL of DMSO (the final concentration of DMSO was 50%) was added to dissolve 0.1 g of Substrate 1, 4 eq of isopropylamine hydrochloride and 1.0 mg of PLP (5'-pyridoxal phosphate) were added, and 0.1 M PB 7.0 was supplemented until the final volume of reaction solution was 1 mL, and then 3 mg of an enzyme or immobilized transaminase prepared from 3 mg of the enzyme was added. It was stirred at 45°C for 16 h. The conversion rate of the system was detected by HPLC, and reaction data was shown in Table 13 below.

**Table 13:**

| Strain number | Immobilization carrier | Conversion rate (%) | Number of repeated uses with activity loss <5% |
|---|---|---|---|
| Wild-type parent | None | 36.2 | 1 |
| | ECR8309 | 19.4 | 4 |
| | ECR8409 | 28.1 | 7 |
| | LX1000HA | 25.7 | 5 |
| | LXEPHA | 26.2 | 6 |
| | LX1000HFA | 14.3 | 3 |
| | ECR8285 | 18.0 | 3 |
| C418Q | None | 58.2 | 1 |
| | ECR8309 | 44.3 | 7 |
| | ECR8409 | 49.6 | 10 |
| | LX1000HA | 53.2 | 9 |
| | LX1000HFA | 48.8 | 7 |
| | ECR8285 | 34.1 | 6 |
| W60Y | None | 51.4 | 1 |
| | ECR8309 | 35.6 | 6 |
| | ECR8409 | 48.6 | 11 |
| | LX1000HA | 47.9 | 12 |
| | LX1000HFA | 39.9 | 6 |
| | ECR8285 | 41.1 | 8 |
| C418Q+V379L | None | 84.2 | 1 |
| | ECR8309 | 79.0 | 18 |
| | ECR8409 | 81.0 | 22 |
| | LX1000HA | 85.1 | 22 |
| | LXEPHA | 82.9 | 18 |
| | ECR8285 | 71.6 | 18 |
| C418Q+V379M | None | 89.7 | 1 |
| | ECR8309 | 78.4 | 18 |
| | ECR8409 | 88.9 | 25 |
| | LX1000HA | 81.9 | 21 |
| | LXEPHA | 68.3 | 20 |
| | ECR8285 | 72.8 | 16 |

### Embodiment 16: Reaction activity detection of covalent immobilized transaminase in 35%~45% methanol aqueous solution

In a 10 mL reaction flask, 0.35-0.45 mL of methanol was added to dissolve 0.1 g of Substrate 1, 4 eq of isopropylamine hydrochloride and 1.0 mg of PLP (5'-pyridoxal phosphate) were added, and 0.1 M PB 7.0 was supplemented until the final volume of reaction solution was 1 mL, and then 10 mg of an enzyme or immobilized transaminase prepared from 10 mg of the enzyme was added. It was stirred at 30°C for 16 h. The conversion rate of the system was detected by HPLC, and reaction data was shown in Table 14 below.

**Table 14:**

| Strain number | Immobilization carrier | Methanol concentration (%) | Conversion rate (%) | Number of repeated uses with activity loss <5% |
|---|---|---|---|---|
| Wild-type parent | None | 35 | 31.7 | 1 |
| | ECR8309 | 35 | 11.6 | 2 |
| | ECR8409 | 35 | 18.9 | 5 |
| | LX1000HA | 35 | 21.8 | 5 |
| | LXEPHA | 35 | 22.1 | 6 |
| | LX1000HFA | 35 | 11.5 | 2 |
| | ECR8285 | 35 | 11.8 | 2 |
| C418Q | None | 35 | 52.7 | 1 |
| | ECR8309 | 35 | 41.1 | 6 |
| | ECR8409 | 35 | 50.8 | 8 |
| | LX1000HA | 35 | 51.0 | 9 |
| | LX1000HFA | 35 | 39.6 | 7 |
| | ECR8285 | 35 | 39.8 | 7 |
| | ECR8409 | 40 | 41.7 | 7 |
| | ECR8409 | 45 | 33.8 | 5 |
| C418Q+V379L | None | 35 | 84.9 | 1 |
| | ECR8309 | 35 | 74.3 | 18 |
| | ECR8409 | 35 | 82.8 | 25 |
| | LX1000HA | 35 | 81.6 | 20 |
| | LXEPHA | 35 | 81.3 | 20 |
| | ECR8285 | 35 | 79.9 | 18 |
| | LX1000HA | 40 | 71.8 | 16 |
| C418Q+V379M | None | 35 | 89.7 | 1 |
| | ECR8309 | 35 | 78.4 | 18 |
| | ECR8409 | 35 | 88.3 | 25 |
| | LX1000HA | 35 | 81.9 | 22 |
| | LXEPHA | 35 | 68.3 | 20 |
| | ECR8285 | 35 | 72.8 | 13 |
| | LX1000HA | 40 | 72.2 | 19 |
| | LXEPHA | 45 | 65.1 | 16 |
| | ECR8285 | 40 | 59.8 | 10 |

### Embodiment 17: Reaction activity detection of covalent immobilized transaminase in organic phase

In a 10 mL reaction flask, 1 mL of water saturated isopropyl acetate was added, then 100 mg of Substrate 1 and 4 eq of isopropylamine were added, and 100 mg of an immobilized transaminase prepared by an enzyme was added. It was stirred at 30°C for 16 h. The conversion rate of the system was detected by HPLC, and reaction data was shown in Table 15 below.

**Table 15:**

| Strain number | Immobilization carrier | Conversion rate (%) | Number of repeated uses with activity loss <5% |
|---|---|---|---|
| Wild-type parent | ECR8409 | >92 | 5 |
| | LX1000HA | >92 | 5 |
| | LXEPHA | >92 | 3 |
| C418Q | ECR8409 | >92 | 12 |
| | LX1000HA | >92 | 12 |
| | LX1000HFA | >92 | 9 |
| | ECR8285 | >92 | 10 |
| W60Y | ECR8409 | >92 | 10 |
| | LX1000HA | >92 | 11 |
| | ECR8285 | >92 | 9 |
| C418Q+V379L | ECR8409 | >92 | 18 |
| | LX1000HA | >92 | 20 |
| | LXEPHA | >92 | 20 |
| | LX1000HFA | >92 | 10 |
| | ECR8285 | >92 | 11 |
| C418Q+V379M | ECR8409 | >92 | 20 |
| | LX1000HA | >92 | 20 |
| | LXEPHA | >92 | 20 |
| | LX1000HFA | >92 | 15 |
| | ECR8285 | >92 | 14 |

### Embodiment 18: Affinity carrier immobilization

5 mL of PB containing 0.4 mg/mL of PLP (50 mM, pH 7.0) was added to a carrier, and 0.1 g of an enzyme was added simultaneously. It was stirred overnight at 20°C and a low speed of 80 rpm. A supernatant was removed, and a precipitate was washed with the buffer solution for 3-4 times. The supernatant was removed, and the precipitate was dried by a nitrogen-blowing or freeze-drying method, and stored at 4°C.

### Embodiment 19: Reaction activity detection of affinity immobilized enzyme in aqueous phase

In a 10 mL reaction flask, 0.6 mL of DMSO (the final concentration of DMSO was 60%) was added to dissolve 0.1 g of Substrate 1, 4 eq of isopropylamine hydrochloride and 1.0 mg of PLP (5'-pyridoxal phosphate) were added, and 0.1 M PB 7.0 was supplemented until the final volume of reaction solution was 1 mL, and then 10 mg of an enzyme or immobilized transaminase prepared from 10 mg of the enzyme was added. It was stirred at 30°C for 16 h. The conversion rate of the system was detected by HPLC, and reaction data was shown in Table 16 below.

**Table 16:**

| Strain number | Immobilization carrier | Conversion rate (%) | Number of repeated uses with activity loss <5% |
|---|---|---|---|
| W60Y | None | 49.2 | 1 |
| | IMAC-Ni | 49.5 | 9 |
| | MIDA-Ni | 44.3 | 9 |
| | MIDA-Cu | 41.1 | 7 |
| C418Q | None | 55.4 | 1 |
| | IMAC-Ni | 50.5 | 16 |
| | MIDA-Ni | 48.2 | 12 |
| C418Q+V379L | None | 83.6 | 1 |
| | IMAC-Ni | 82.9 | 20 |
| | MIDA-Ni | 80.3 | 19 |
| | MIDA-Cu | 79.9 | 18 |
| C418Q+V379M | None | 88.8 | 1 |
| | IMAC-Ni | 79.3 | 20 |
| | MIDA-Ni | 83.4 | 20 |
| | MIDA-Cu | 72.9 | 18 |

### Embodiment 20: Reaction activity detection of affinity immobilized transaminase in organic phase

In a 10 mL reaction flask, 1 mL of water saturated methyl tert-butyl ether was added, then 100 mg of Substrate 1 and 4 eq of isopropylamine were added, and 100 mg of an immobilized transaminase prepared by an enzyme was added. It was stirred at 30°C for 16 h. The conversion rate of the system was detected by HPLC, and reaction data was shown in Table 17 below.

**Table 17:**

| Strain number | Immobilization carrier | Conversion rate (%) | Number of repeated uses with activity loss <5% |
|---|---|---|---|
| W60Y | IMAC-Ni | >92 | 10 |
| | MIDA-Ni | >92 | 10 |
| C418Q | IMAC-Ni | >92 | 10 |
| | MIDA-Ni | >92 | 10 |
| C418Q+V379L | IMAC-Ni | >92 | 20 |
| | MIDA-Ni | >92 | 19 |
| | MIDA-Cu | >92 | 16 |
| C418Q+V379M | IMAC-Ni | >92 | 20 |
| | MIDA-Ni | >92 | 20 |
| | MIDA-Cu | >92 | 18 |

### Embodiment 21: Continuous reaction of immobilized enzyme transaminase in packed bed

An immobilized enzyme of 382 g of a mutant C418Q+V379M immobilized onto an LX1000HA carrier was filled into a reactor, the column volume (CV) was 750 mL, and a plunger pump was used to inject 2 CV buffer (0.1 M PB 7.0, containing 5 mg/mL of PLP, and 2 M isopropylamine hydrochloride) into a packed bed. Reaction solution (0.5 M Substrate 1, 2 M isopropylamine hydrochloride, 5 mg/mL PLP, and 40% MeOH) prepared was injected into the packed bed with the plunger pump, in a water bath at 37°C, the retention time was about 200 min, the conversion rate was >98%, and the space-time yield (STY) reached 3.6 mol/L/day. It was continuously operated for 400 h, and the conversion rate was not decreased.

### Embodiment 22: Continuous reaction of PEI cross-linked immobilized enzyme activated by glutaraldehyde in packed bed

An immobilized enzyme of 76 g of a mutant C418Q+V379M immobilized onto an LX1000HA carrier was filled into a reactor, the column volume (CV) was 150 mL, and a plunger pump was used to inject 2 CV buffer (0.1 M PB 7.0, containing 5 mg/mL of PLP, and 2 M isopropylamine hydrochloride) into a packed bed. Reaction solution (0.5 M Substrate 1, 2 M isopropylamine hydrochloride, 5 mg/mL PLP, and 40% MeOH) prepared was injected into the packed bed with the plunger pump, in a water bath at 37°C, the retention time was about 100 min, the conversion rate was >98%, and STY reached 7.3 mol/L/day. It was continuously operated for 400 h, and the conversion rate was not decreased.

From the above description, it may be seen that the above embodiments of the present disclosure achieve the following technical effects: the mutant with the improved activity, stability, and tolerance to temperature, pH, and organic solvent is obtained by directed evolution screening, not only the usage amount of the enzyme in the production applications is reduced, but also the possibility of preparing various immobilized enzymes is greatly improved. In addition, by immobilizing the above transaminase after the directed evolution (self-crosslinking or covalent-binding with the carrier) in the present application, the applications of the immobilized transaminase in the aqueous phase reaction and the organic phase reaction may be achieved, so that the enzyme is easily separated from the reaction system and the emulsification phenomenon caused by the residual enzyme protein during the reaction post-treatment process is reduced. At the same time, the immobilized transaminase mutant may withstand various extreme environments with low activity loss and high reuse times; thereby the continuous transamination reactions of Substrate 1 and Substrate 2 are achieved.

The above are only preferred embodiments of the present disclosure and are not intended to limit the present disclosure. For those skilled in the art, the present disclosure may have various modifications and variations. Any modifications, equivalent replacements, improvements and the like made within the spirit and principles of the present disclosure shall be included within the scope of protection of the present disclosure.

## Claims

1. A transaminase mutant, wherein the transaminase mutant comprising: a protein having the amino acid sequence of SEQ ID NO: 1 with a mutation of one or more amino acids, wherein the mutation comprises any one or more of the group consisting of: C418Q, C418W, W60Y, Y168A, V379W, V379L, V379M, W60Y+Y168A, W60Y+V379W, W60Y+V379L, W60Y+V379M, W60Y+C418Q, W60Y+C418W, Y168A+V379W, Y168A+V379L, Y168A+V379M, Y168A+C418Q, Y168A+C418W, V379W+C418Q, V379W+C418W, V379L+C418Q, V379L+C418W, V379M+C418Q, V379M+C418W, W60Y+Y168A+V379W, W60Y+Y168A+V379L, W60Y+Y168A+V379M, W60Y+Y168A+C418Q, W60Y+Y168A+C418W, W60Y+V379W+C418Q, W60Y+V379W+C418W, W60Y+V379L+C418Q, W60Y+V379L+C418W, W60Y+V379M+C418Q, W60Y+V379M+C418W, Y168A+V379W+C418Q, Y168A+V379W+C418W, Y168A+V379L+C418Q, Y168A+V379L+C418W, Y168A+V379M+C418Q, Y168A+V379M+C418W, W60Y+Y168A+V379W+C418Q, W60Y+Y168A+V379L+C418Q, W60Y+Y168A+V379M+C418Q, W60Y+Y168A+V379W+C418W, W60Y+Y168A+V379L+C418W or W60Y+Y168A+V379M+C418W;.
alternatively, the amino acid sequence of the transaminase mutant has a mutation site in the mutated amino acid sequence, and has more than 85% of identity with the mutated amino acid sequence, and has the transaminase activity.

2. The transaminase mutant according to claim 1, wherein the amino acid sequence of the transaminase mutant has more than 90%, preferably more than 95%, and more preferably more than 99% of the identity with the mutated amino acid sequence, and has the transaminase activity.

3. The transaminase mutant according to claim 1 or 2, wherein the transaminase mutant is derived from *Chromobacterium violaceum.*

4. A DNA molecule, wherein the DNA molecule encodes the transaminase mutant of any one of claims 1-3.

5. A recombinant plasmid, wherein the recombinant plasmid is linked with the DNA molecule of claim 4.

6. An immobilized transaminase, wherein the immobilized transaminase comprises the transaminase mutant of any one of claims 1-3.

7. The immobilized transaminase according to claim 6, wherein the immobilized transaminase is a cross-linked immobilized enzyme aggregate of the transaminase mutant;
preferably, the cross-linked immobilized enzyme aggregate is an immobilized enzyme aggregate cross-linked by glutaraldehyde;
preferably, the cross-linked immobilized enzyme aggregate is an immobilized enzyme aggregate cross-linked by glutaraldehyde-activated PEl;
and more preferably, the cross-linked immobilized enzyme aggregate is an immobilized enzyme aggregate cross-linked by cofactor-activated PEI via glutaraldehyde.

8. The immobilized transaminase according to claim 6, wherein the immobilized transaminase is an immobilized enzyme formed by binding the transaminase mutant to a carrier;
preferably, the carrier is a cross-linked polymer resin carrier;
preferably, the resin carrier comprises a macroporous adsorption resin sphere, an amino resin sphere or an epoxy resin sphere;
preferably, the macroporous adsorption resin sphere has a matrix of polystyrene;
more preferably, the macroporous adsorption resin sphere comprises NKA9, LXEP120, AB-8, ECR8806, XAD-7 or D3520;
preferably, the amino resin sphere is a cross-linked polymer resin sphere with an amino functional group;
more preferably, a functional group of the amino resin sphere is an amino functional group matrix having a chain length C1-C4 or C5-C10;
more preferably, the functional group of the amino resin sphere is an amino functional group having a carbon chain arm of chain length C2 or C6;
more preferably, the amino resin sphere is a polymethacrylate resin sphere, a polystyrene resin sphere or a methacrylate-styrene copolymer resin sphere;
more preferably, the amino resin sphere comprises Lifetech^{™}ECR8309, Lifetech^{™}ECR8409, Seplite^{®}LX1000HA or Seplite^{®}LXEPHA;
preferably, the epoxy resin sphere is a polymer resin sphere with an epoxy functional group;
more preferably, the epoxy resin sphere has the epoxy functional group with a carbon chain arm of a C2-C8 length;
more preferably, the epoxy resin sphere is a polymethacrylate resin sphere with an epoxy functional group, a polystyrene resin, or a resin sphere of a methacrylate and styrene copolymer;
more preferably, the epoxy resin sphere is a polymethacrylate resin sphere, a polystyrene resin sphere or a methacrylate-styrene copolymer resin, having the epoxy functional group;
more preferably, the epoxy resin sphere comprises Seplite^{®}LXHFA001 or Lifetech^{™} ECR8285.

9. The immobilized transaminase according to claim 6, wherein the immobilized transaminase is an affinity carrier-immobilized enzyme formed by affinity adsorption between the transaminase mutant and a metal affinity adsorption-type carrier;
preferably, the carrier is a resin sphere having a polymethylacrylic acid matrix;
more preferably, the resin sphere comprises an NTA or IDA ligand;
more preferably, the resin sphere chelates the metal ion ligand at the terminal;
further preferably, the metal ion ligand comprises Ni²⁺, Co²⁺, Cu²⁺ or Fe³⁺;
further preferably, the carrier comprises IMAC-Ni or MIDA-Ni;
further preferably, the carrier comprises Bio-Rad Profinity^{™} IMAC Resin, Purolite Chromalite^{™} MIDA/M/Ni, Purolite Chromalite^{™} MIDA/M/Co, Purolite Chromalite^{™} MIDA/M/Cu or Purolite Chromalite^{™} MIDA/M/Fe.

10. A method for producing chiral amine with a transaminase, comprising a step of performing a catalytic transamination on a ketone compound and an amino donor with the transaminase, wherein the transaminase is the transaminase mutant of any one of claims 1-3 or the immobilized transaminase of any one of claims 6-9.

11. The method according to claim 10, wherein the method is a batch reaction or a continuous reaction;
preferably, the batch reaction or the continuous reaction is performed in an aqueous phase or an organic phase;
preferably, conditions for performing the reaction in the aqueous phase are that: an organic solvent as a co solvent, which is mutually soluble with water and accounts for not greater than 70% by volume of the total system, is added to an aqueous solution for catalytic reaction; further, the organic solvent is methanol, ethanol or dimethyl sulfoxide;
preferably, conditions for performing the reaction in the organic phase are that: a catalytic reaction is performed in a water-saturated organic solvent which is not mutually soluble with water, further, the catalytic reaction is performed in a water-saturated methyl tert-butyl ether solution or a water-saturated isopropyl acetate solution;
preferably, the immobilized transaminase is the immobilized transaminase of any one od claims 6-9, and the immobilized transaminase is packed into a tube to achieve a packed bed continuous reaction;
preferably, a continuous use time of the immobilized enzyme is not less than 400 h;
preferably, a space-time yield of the immobilized enzyme is 3.6-7.2 mol/L/day.

12. The method according to claim 11, wherein the ketone compound is 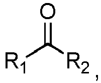 wherein, R₁ and R₂ are each independently C1-C8 alkyl, C5-C10 cycloalkyl or C6-C10 aryl, or R₁ and R₂ form a C5-C10 heterocyclic radical or a C5-C10 carbocyclic radical together with a C on carbonyl, and heteroatoms in the C5-C10 heterocyclic radical are each independently selected from at least one of N, O and S; the aryl in the C6-C10 aryl, the carbocyclic radical in the C5-C10 carbocyclic radical or the heterocyclic radical in the C5-C10 heterocyclic radical are each independently unsubstituted or substituted by at least one radical of halogen, alkoxy or alkyl;
preferably, the R₁ and R₂ are each independently C5-C10 heterocycloalkane, and heteroatoms in the C5-C10 heterocycloalkane are each independently selected from at least one of N, O and S; the carbocyclic radical in the C5-C10 heterocycloalkane is unsubstituted or substituted by at least one radical of halogen, alkoxy or alkyl;
preferably, the ketone compound is 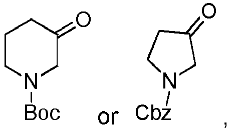 wherein a product of the transamination is

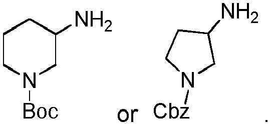

13. A production method of an immobilized transaminase, wherein the production method comprises a step of immobilizing a transaminase mutant, wherein the transaminase mutant is the transaminase mutant of any one of claims 1-3.

14. The production method according to claim 13, wherein the step of immobilizing the transaminase mutant comprises: crosslinking and immobilizing the transaminase mutant to form an immobilized enzyme aggregate;
preferably, crosslinking and immobilizing the transaminase mutant to form an immobilized enzyme aggregate comprises:
crosslinking an enzyme precipitated by a precipitant with glutaraldehyde to form the immobilized enzyme aggregate; or
crosslinking an enzyme precipitated by a precipitant with a glutaraldehyde-activated PEI to form the immobilized enzyme aggregate; or
crosslinking the transaminase mutant with a cofactor-activated PEI via glutaraldehyde to form the immobilized enzyme aggregate.

15. The production method according to claim 13, wherein the step of immobilizing the transaminase mutant comprises: linking the transaminase mutant with a carrier to form an immobilized enzyme;
preferably, the carrier is a cross-linked polymer resin sphere;
preferably, the resin carrier comprises a macroporous adsorption resin sphere, an amino carrier sphere or an epoxy carrier sphere;
preferably, the macroporous adsorption resin sphere has a matrix of polystyrene;
more preferably, the macroporous adsorption resin sphere comprises NKA9, LXEP120, AB-8, ECR8806, XAD-7 or D3520;
preferably, the amino resin sphere is a cross-linked polymer resin sphere with an amino functional group;
more preferably, the functional group of the amino resin sphere is an amino functional group matrix having a carbon chain arm of chain length C2 or C6;
more preferably, the amino carrier comprises a polymethacrylate resin sphere, a polystyrene resin sphere or a methacrylate-styrene copolymer resin sphere;
more preferably, the amino resin sphere comprises Lifetech^{™}ECR8309, Lifetech^{™}ECR8409, Seplite^{®}LX1000HA or Seplite^{®}LXEPHA;
preferably, the epoxy resin sphere is a polymer resin sphere with an epoxy functional group;
more preferably, the epoxy resin sphere has an epoxy functional group with a carbon chain arm of a C2-C8 length;
more preferably, the epoxy resin sphere is a polymethacrylate resin sphere, a polystyrene resin sphere or a methacrylate-styrene copolymer resin, having the epoxy functional group;
more preferably, the epoxy resin sphere comprises Seplite^{®}LXHFA001 or Lifetech^{™}ECR8285.

16. The production method according to claim 13, wherein the step of immobilizing the transaminase mutant comprises the followings:
performing affinity adsorption on the transaminase mutant and a carrier to form an affinity immobilized enzyme;
preferably, the transaminase mutant is co-incubated with the carrier to obtain the affinity immobilized enzyme;
preferably, transaminase mutant has a plurality of His-tags;
preferably, the carrier is a resin sphere having a polymethylacrylic acid matrix;
more preferably, the resin sphere comprises an NTA or IDA ligand;
more preferably, the resin sphere chelates the metal ion ligand at the terminal;
further preferably, the metal ion ligand comprises Ni²⁺, Co²⁺, Cu²⁺ or Fe³⁺;
further preferably, the resin sphere comprises IMAC-Ni or MIDA-Ni;
further preferably, the resin sphere comprises Bio-Rad Profinity^{™} IMAC Resin, Purolite Chromalite^{™} MIDA/M/Ni, Purolite Chromalite^{™} MIDA/M/Co, Purolite Chromalite^{™} MIDA/M/Cu or Purolite Chromalite^{™} MIDA/M/Fe.
